# EUROPÄISCHE PATENTSCHRIFT

(11) **EP 3 170 427 B1**
(45) Veröffentlichungstag und Bekanntmachung des Hinweises auf die Patenterteilung: **04.07.2018**
(21) Anmeldenummer: 16193778.4
(22) Anmeldetag: 13.10.2016
(51) Int. Cl.: A47B 47/04, A61L 9/00, A47B 95/00

(54) **HALTESTRUKTUR ZUR AUFNAHME VON HOLZSPÄNEN UND REGALANORDNUNG ZUR AUFNAHME VON HALTESTRUKTUREN**
SUPPORT STRUCTURE FOR RECEIVING WOOD CHIPS AND SHELF ASSEMBLY FOR RECEIVING SUPPORT STRUCTURES
STRUCTURE SUPPORT DE RÉCEPTION DE COPEAUX DE BOIS ET ENSEMBLE D'ÉTAGÈRES DESTINÉ À LOGER LES STRUCTURES DE SUPPORT

(30) Priorität: 28.10.2015 DE 102015118410
(43) Veröffentlichungstag der Anmeldung: 24.05.2017
(73) Patentinhaber: Rapp, Klaus-Dieter, 78730 Lauterbach (DE)
(72) Erfinder: Rapp, Klaus-Dieter, 78730 Lauterbach (DE)
(74) Vertreter: Herrmann, Jochen

(56) Entgegenhaltungen:
- FR-A- 847 748
- US-A1- 2004 140 239
- US-A1- 2007 007 224
- US-B1- 6 669 036

## Beschreibung

Die Erfindung betrifft eine Haltestruktur zur Aufnahme von Holzspänenund ein Wandelement zur Aufstellung auf einer Aufstellfläche, mit einem oder mehreren Regalfächern jeweils zur wechselbaren Aufnahme einer oder mehrerer dieser Haltestrukturen.

Aus dem Stand der Technik ist bekannt, Holz beispielsweise bei der Möbelherstellung, im Hausbau und zur Herstellung von Wandvertäfelungen nicht nur als Werkstoff zu benutzen, sondern vielmehr auch den aromatischen Duft des Holzes bewusst zur Luftaromatisierung im Wohn- und Schlafbereich zu verwenden.

In der Wikipedia Enzyklopädie wird beschrieben, dass insbesondere aus der Zirbelkiefer, auch Arbe, Arve, Zirbe oder Zirbel genannt, ein sehr aromatisch duftendes Holz gewonnen wird, das insbesondere als Möbel- und Schnitzholz Verwendung findet. In Südtirol wurden schon im 18. Jahrhundert verschiedenste Extrakte der Zirbel als Heilmittel verwendet. Heutzutage hat sich eine Form der Wellnessbehandlung mit verschiedenen Bestandteilen der Zirbel entwickelt. In der Schweiz werden beispielsweise Arvenkissen hergestellt, in Bayern Zirbenkissen und in Österreich Zirbenrollen mit speziell gehobelten Spänen des Zirbenholzes als Füllmaterial. Die aus den Spänen über lange Zeit ausströmenden Düfte mit dem typischen Zirbenduft sollen für einen tiefen und gesunden Schlaf sorgen. Auch Betten aus Zirbenholz wird nachgesagt, dass sie den Schlaf verbessern.

Das Holz der Zirbelkiefer wird vor allem wegen der lebhaften Zeichnung im Innenausbau für Täfelungen sowie als Möbelholz für Bauernküchen und Schlafzimmer genutzt.

Die spezielle Optik des Zirbelholzes insbesondere als Wandvertäfelung trifft nicht den Geschmack jedes Betrachters. Bei der Wohn- oder Schlafraumgestaltung würden sich zwar viele Menschen den Duft und die positiven Eigenschaften einer entsprechenden Luftaromatisierung durch Zirbelholz wünschen, haben aber bezüglich der optischen Erscheinung bzw. des Wohnraumdesigns bestimmte Vorstellungen, die sich nicht mit Zirbelholz als Werkstoff ohne weiteres realisieren lassen.

US 2004/0140239 A1 offenbart einen Bevorratungsbehälter in Form eines Regals. Dabei sind gegenüberliegend zwei vertikale Seitenwände, sowie ein Boden und eine Deckwand vorgesehen. Die Seitenwände und die Deckwand sind aus streifenförmigen Bambusbrettern zusammengesetzt, sodass sich rasterförmig Durchbrechungen ergeben.

US 2007/0007224 A1 offenbart einen Korb, wobei die Seitenwände des Korbs aus ringförmigen Elementen zusammengesetzt sind.

Es ist Aufgabe der Erfindung, ein gemäß dem gewünschten Design gestaltbares Wandelement oder ein Wandelement anzugeben, bei dem im Wohnraum von den positiven Eigenschaften des Zirbelholzes profitiert werden kann.

Die Aufgabe der Erfindung wird durch eine Haltestruktur zur Aufnahme von Holzspänen gemäß den Merkmalen des Patentanspruchs 1 gelöst. Vorteilhafte Weiterbildungen sind jeweils in den Unteransprüchen beschrieben.

Demnach weist die Haltestruktur zur Aufnahme von Holzspänen einen durch Außenwände umschlossenen Aufnahmeraum für die Holzspäne auf, wobei die Außenwände zumindest diffusionsoffene Teilbereiche zum Entweichen von aus den Holzspänen austretenden Düfte aufweisen. Mit dieser Haltestruktur werden die aus spanender Bearbeitung von Holz erzeugten Holzspäne bzw. kleine Holzteile sicher im Aufnahmeraum gehalten und haben durch die diffusionsoffenen Teilbereiche die Möglichkeit, die aromatischen Duftstoffe der im Holz enthaltenden Inhaltsstoffe nach Außen, beispielsweise in den Wohnraum abzugeben.

Zumindest ein Teil der Außenwände besteht aus einem Gitter oder Geflecht und ein anderer Teil der Außenwände ist aus Holz gefertigt. Das Geflecht verhindert, dass die Holzspäne und kleinere Holzteilchen aus der Haltestruktur herausfallen und ermöglicht ein weitgehend ungehindertes Ausströmen der im Holz enthaltenen Duftstoffe. Durch die Holzwände erlangt die Haltestruktur eine besondere Stabilität. Insbesondere dann, wenn das für die Außenwände verwendete Holz ebenfalls aromatische Duftstoffe freisetzt wird der Aromaeffekt der Holzspäne noch verstärkt. Das Geflecht kann beispielsweise ein Kunststoffgitter oder ein Drahtgitter oder dergleichen sein.

Eine besonders stabile Haltestruktur wird dadurch geschaffen, dass der Aufnahmeraum im Wesentlichen quaderförmig aufgebaut ist, zwei sich gegenüberliegende Außenwände als Holzwände ausgebildet sind und die übrigen vier Außenwände aus dem Geflecht bestehen.

Um die Haltestruktur noch weiter zu verstärken und die zwei sich gegenüberliegenden Holzwände in einem festen Abstand zueinander zu halten, sind die sich gegenüberliegenden Holzwände durch vier an den Kantenbereichen angeordnete Streben zueinander beanstandet, wobei das Geflecht zumindest teilweise umläufig über die Streben gespannt ist. Mit diesem Aufbau wird außerdem der Geflechtaufbau weiter stabilisiert und es wird verhindert, dass sich das Geflecht in Richtung auf den Aufnahmeraum oder nach außen übermäßig verformt.

Die sich gegenüberliegenden Holzwände können jeweils zumindest mit einem Wandteil an einer Seite über den dazwischenliegenden Aufnahmeraum hervorstehen, wobei die sich gegenüberliegenden vorstehenden Wandteile jeweils zumindest einen Ausschnitt aufweisen können, der sich bis zur äußeren Begrenzung des Aufnahmeraumes erstreckt. Somit wird erreicht, dass die Holzwände selber diffusionsoffene Teilbereiche aufweisen, so dass selbst dann, wenn die Haltestruktur auf einer ebenen Fläche oder an einer ebenen Wand an den Kanten der Außenwände aufliegend platziert ist, die von den im Aufnahmeraum befindlichen Holzspänen ausgehenden Duftstoffe durch die Geflechtstruktur und die Aussparungen hindurch beispielsweise in den Wohnraum ausströmen können.

In besonders einfacher Ausgestaltung kann das Geflecht jeweils in die Ausschnitte hineinreichen und dort befestigt sein. Damit wird eine weitere Vereinfachung des Aufbaus und eine Möglichkeit geschaffen, das Geflecht auf einfache Weise an den Außenwänden zu befestigen.

Die Aufgabe der Erfindung wird gemäß den Merkmalen des Patentanspruchs 4 auch durch ein Wandelement mit einer Regalanordnung zur Aufstellung auf einer Aufstellfläche, mit einem oder mehreren Regalfächern jeweils zur wechselbaren Aufnahme einer oder mehrerer Haltestrukturen gemäß Patentanspruch 1 gelöst. Vorteilhafte Weiterbildungen sind jeweils in den Unteransprüchen beschrieben.

Die Haltestrukturen könne beispielsweise im Wohnraum frei oder an beliebigen Orten aufgestellt werden, um die Düfte zu verteilen, sind aber insbesondere auch dazu ausgebildet, in einer speziell gestalteten Regalanordnung wechselbar eingesetzt zu werden.

Gemäß einem Grundgedanken der Erfindungen kann die Regalanordnung zwei sich in einem Abstand zueinander gegenüberliegend angeordneten Regalböden aufweisen, die ein dazwischenliegendes Regalfach zur Aufnahme der einen oder mehrerer Haltestrukturen definieren. Mit dieser Anordnung können je nach Länge der Regalböden und der Größe der Haltestrukturen ein oder mehrere Haltestrukturen nebeneinander auf einem Regalboden innerhalb des Regalfachs angeordnet werden. Insbesondere können die Haltestrukturen von der Längsseite der Regalböden in das Regalfach nebeneinander eingesetzt werden. Alternativ können die Haltestrukturen an der Schmalseite hintereinander in das Regalfach eingeschoben werden. Je nach Orientierung der Regalböden und somit des Regalfachs können die Haltestrukturen somit nebeneinander oder übereinander angeordnet werden.

Ein besonders stabiler Aufbau der Regalanordnung lässt sich dadurch erreichen, dass zwischen den zueinander gegenüberliegend angeordneten Regalböden in einem Winkel zu diesen ein oder mehrere Unterteilungsböden angeordnet sind, die das Regalfach in zwei oder mehrere Teilfächer jeweils zur Aufnahme einer oder mehrerer Haltestrukturen teilt. Insbesondere bei langen Regalböden und der Anordnung von mehreren Regalfächern über- oder nebeneinander wird durch die Unterteilungsböden eine erhöhte Stabilität der Regalanordnung erreicht. Die dadurch definierten Teilfächer sind für die Aufnahme einer oder mehrerer Haltestrukturen geeignet. Gemäß einer besonders stabilen und die Haltestrukturen sicher haltenden Ausführungsform können die Teilfächer so ausgebildet sein, dass jeweils eine Haltestruktur passgenau in einem Teilfach aufgenommen werden kann. Je nach Skalierung eines Teilfachs und der eingesetzten Haltestruktur kann die Regalanordnung auch nur aus diesem einen Teilfach bestehen, das aus zwei sich gegenüberliegenden Regalböden und zwei sich gegenüberliegenden Unterteilungsböden besteht.

Gemäß einer erfindungsgemäßen Weiterbildung können die Mehrzahl von Regalböden und die Mehrzahl von Unterteilungsböden eine Gitteranordnung mit einer Mehrzahl von rasterförmig angeordneten Teilfächern bilden. Damit kann erreicht werden, dass eine Mehrzahl von Haltestrukturen in räumlicher Nähe zueinander gehalten werden kann. Zudem ist die rasterförmige Anordnung besonders verwindungssteif und weist eine erhöhte Tragkraft auf.

Eine noch weitere Verbesserung des Ausströmungsvermögens der in der Haltestruktur gehaltenen Holzspäne lässt sich dadurch erreichen, dass zumindest ein Teil der Regalböden oder Unterteilungsböden am Kantenbereich zumindest einen Ausschnitt aufweisen, der sich in Richtung Mitte der Regalböden oder Unterteilungsböden erstreckt. Mit dieser Maßnahme wird erreicht, dass selbst dann, wenn die Regalanordnung flächig abgedeckt wird, die Duftstoffe aus den auf den Regalböden platzierten Haltestrukturen zwischen Regalanordnung und Abdeckung abströmen können.

Gemäß einer bevorzugten erfindungsgemäßen Weiterbildung kann zumindest ein Teil der die Teilfächer definierenden Regalböden und Unterteilungsböden am Kantenbereich zumindest eines Teils der Teilfächer jeweils einen eingezogenen Ausschnitt aufweisen, der sich vom Kantenbereich in Richtung Teilfachmitte erstreckt, wobei die jeweiligen Kontaktbereiche zwischen Regalboden und Unterteilungsboden ausschnittfrei sind. Mit dieser Maßnahme wird erreicht, dass die Holzspäne in einer Haltestruktur, die in einem Teilfach eingesetzt ist, und deren Duftstoffe durch die Ausschnitte in den angrenzenden Regalböden und den Unterteilungsböden optimal ausströmen können. Dabei wird die Haltestruktur stabil in dem Teilfach gehalten, da die Kontaktbereiche, in denen den Regalböden mit den Unterteilungsböden zusammengefügt sind, nicht Teil der Ausschnitte sind, sondern vielmehr Auflageflächen für die Haltestruktur, insbesondere an den Eckbereichen der Außenwände bilden. Es kommt somit an den sich gegenüberstehenden Kantenbereichen der Außenwände und der Regalböden oder Unterteilungsböden zu keinem dichtenden Abschluss. Dieser wird durch die Ausschnitte verhindert.

Gemäß einer besonders bevorzugten Ausführungsform der Erfindung lässt sich zumindest einseitig eine Wandverkleidung an der Regalanordnung anbringen, die zumindest einen Teilbereich der Regalanordnung abdeckt. Diese Wandverkleidung dient zum einen als optische Abdeckung der Regalanordnung und verhindert, dass die eingesetzten Haltestrukturen von außen sichtbar sind. Zum anderen dient die Wandverkleidung dazu, aus der Regalanordnung ein Wandelement zu machen, insbesondere wenn beidseitig Wandelemente angebracht sind. Die Regalanordnung dient somit als Stützgestell für eine Wandverkleidung oder aber als Stützgestell für eine freistehende Wand. Die Wandverkleidung kann dabei so ausgestaltet sein, dass sie frei dekorierbar, z.B. verputzbar ist, um dem Design einer Zimmerwand zu genügen. Die Wandverkleidung kann aber auch dampfdurchlässig ausgestaltet sein, um die aromatischen Düfte der Holzspäne hindurchzulassen. Weiterhin kann die Wandverkleidung auch aus derselben Holzart bestehen wie die Holzspäne.

In vorteilhafter Weiterbindung kann an zumindest einem Teil der Kontaktbereiche, in denen den Regalböden mit den Unterteilungsböden zusammengefügt sind, zumindest eine Abstandslatte beispielsweise durch eine Schraubverbindung angebracht sein, welche die Wandverkleidung in einem Abstand zu den Regalböden und Unterteilungsböden hält. Die Abstandslatte oder eine Mehrzahl von Abstandslatten beabstanden die Wandverkleidung von Kantenbereichen der Regalböden und Unterteilungsböden. Durch die Ausschnitte in den Regalböden und den Unterteilungsböden können die Düfte auch die Abstandslatten unterströmen. Die Wandverkleidung kann dabei beispielsweise durch eine Schraubverbindung direkt an den Abstandslatten befestigt sein.

Um im Bodenbereich beispielsweise eines Wohnraums einen Luftaustausch zwischen der Umgebung und den in den Regalfächern angeordneten Haltestrukturen zu ermöglich kann zwischen der Aufstellfläche und der Wandverkleidung eine zur Wandverkleidung beabstandete Sockelblende und im Abstandsbereich zwischen der Sockelblende und der Wandverkleidung ein Zuluftgitter angeordnet sein. Zwischen der Sockelbelende und der Wandverkleidung kann Luft aus der Umgebung durch das luftdurchlässige Zuluftgitter in das Innere der Regalstruktur einströmen, um die aromatischen Düfte aus den Holzspänen aufzunehmen und weiter zu transportieren.

Um im Deckenbereich beispielsweise eines Wohnraums einen Luftaustausch zwischen der Umgebung und den in den Regalfächern angeordneten Haltestrukturen zu ermöglich kann zwischen der Wandverkleidung und einer oberhalb der Regalanordnung angeordneten Deckenfläche ein Abluftgitter angeordnet sein, wobei sich die Wandverkleidung zwischen dem Zuluftgitter und dem Abluftgitter erstreckt. Mit dieser Maßnahme wird erreicht, dass die durch das bodennahe Zuluftgitter einströmende Luft die aromatischen Duftstoffe aus den Holzspänen aufzunimmt und weiter durch das Abluftgitter in den Wohnraum transportiert. Durch eine Art Kamineffekt wird eine Luftzirkulation vom Zuluftgitter zum Abluftgitter erzeugt, die wirkungsvoll die Duftstoffe in den Wohnraum ausströmen lässt.

Damit die Luftzirkulation hinter der Wandabdeckung nicht behindert wird, kann sich gemäß einer weiteren vorteilhaften Ausgestaltung die zumindest eine Abstandslatte von der Aufstellfläche in Richtung Deckenfläche erstrecken. Da die Luftzirkulation in Richtung vom Zuluftgitter zum Abluftgitter erfolgt, behindern die Abstandslatten den Transport der Duftstoffe nicht.

In vorteilhafter Weiterbindung kann in einem Abschnitt der Sockelblende ein Einschubfach für einen nach oben offenen Wasserbehälter ausgebildet sein. Da die Holzspäne sehr hygroskopisch sind und durch Feuchtigkeitsaufnahme die Holzspäne vermehrt zur Abgabe der Duftstoffe angeregt werden, wird durch diese einfache Maßnahme eine fortlaufende Regeneration der Holzspäne im Hinblick auf die Duftstoffproduktion gewährleistet. Trockenes Holz riecht nicht so stark wie feuchtes Holz. Mittels des Einschubfaches kann der Wasserbehälter von Zeit zu Zeit aus dem bodennahen Bereich der Regalanordnung herausgezogen und neu befüllt werden.

Ein besonderer dekorativer Effekt wird dadurch erreicht dass in der Wandverkleidung zumindest eine Aussparung ausgebildet ist, die zumindest ein Teilfach freigibt. Durch die Aussparung hindurch kann das Teilfach dann beispielsweise dazu genutzt werden, einen dekorativen Gegenstand hineinzustellen.

Die Erfindung wird nachfolgend anhand von in den Zeichnungen dargestellten Ausführungsbeispielen näher erläutert.

Es zeigen:
- Figur 1: in schematischer und perspektivischer Frontalansicht eine Ausführungsform einer Haltestruktur;
- Figur 2: in schematischer und perspektivischer Frontalansicht eine Ausführungsform einer auf einer Aufstellfläche aufgestellten Regalanordnung;
- Figur 3: in schematischer und perspektivischer Frontalansicht die in der Figur 2 gezeigte Regalanordnung mit vier in der Figur 1 gezeigten Haltestrukturen, die in vier Teilfächer eingesetzt sind;
- Figur 4: in schematischer und perspektivischer, teilweise geschnittener Frontalansicht die in der Figur 3 gezeigte Regalanordnung zusätzlich mit zwei an der Vorderseite angebrachten Abstandslatten;
- Figur 5: in schematischer und perspektivischer, teilweise geschnittener Frontalansicht die in der Figur 4 gezeigte Regalanordnung zusätzlich mit zwei an der Rückseite angebrachten Abstandslatten;
- Figur 6: in schematischer und perspektivischer, teilweise geschnittener Frontalansicht die in der Figur 5 gezeigte Regalanordnung zusätzlich mit einer an der Rückseite angebrachten Wandverkleidung;
- Figur 7: in schematischer und perspektivischer, teilweise geschnittener Frontalansicht die in der Figur 6 gezeigte Regalanordnung zusätzlich mit einer an der Vorderseite angebrachten Wandverkleidung;
- Figur 8: in schematischer und perspektivischer, teilweise geschnittener Frontalansicht die in der Figur 7 gezeigte Regalanordnung zusätzlich mit einer an der Vorderseite unterhalb der Wandverkleidung angebrachten Sockelblende und einem zwischen Wandverkleidung und Sockelblende angebrachten Zuluftgitter;
- Figur 9: in schematischer und perspektivischer, teilweise geschnittener Frontalansicht die in der Figur 8 gezeigte Regalanordnung zusätzlich mit einem an der Vorderseite an der Sockelblende angeordneten Einschubfach mit Wasserbehälter;
- Figur 10: in schematischer und perspektivischer, teilweise geschnittener Frontalansicht die in der Figur 9 gezeigte Regalanordnung zusätzlich mit einem an der Vorderseite oberhalb der Wandverkleidung zwischen der Wandverkleidung und einer Deckenfläche angebrachten Abluftgitter; und
- Figur 11: in schematischer und perspektivischer, teilweise geschnittener Frontalansicht die in der Figur 10 gezeigte Regalanordnung zusätzlich mit einer an der Vorderseite in der Wandverkleidung ausgebildeten Aussparung.

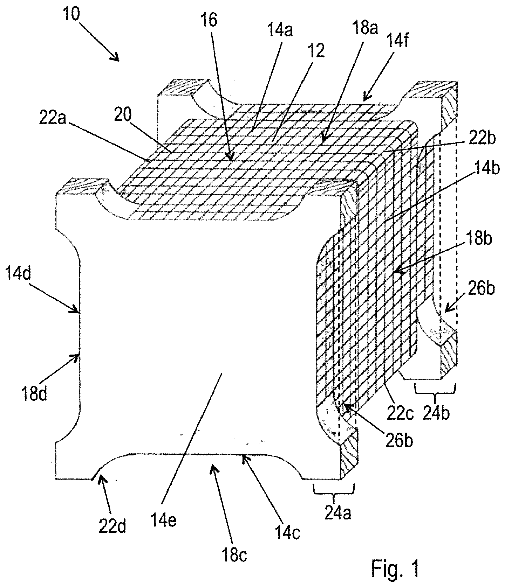

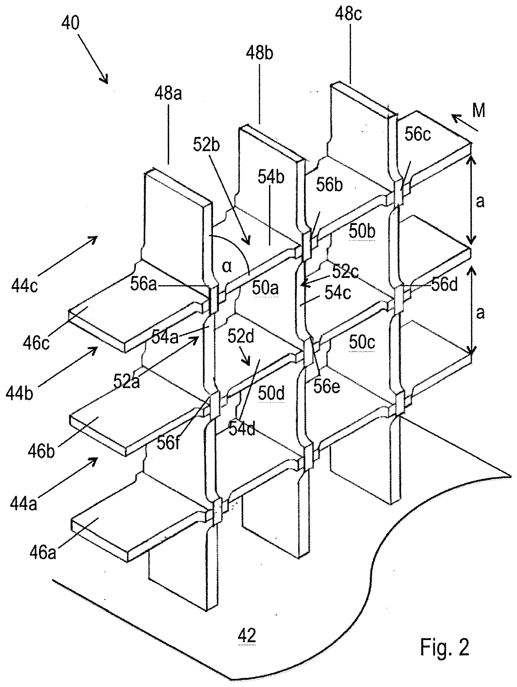

Die Figur 1 zeigt in schematischer und perspektivischer Frontalansicht eine Ausführungsform einer Haltestruktur 10. Die Haltestruktur 10 hat eine im Wesentlichen quaderförmige Grundform und weist einen durch Außenwände 14a, 14b, 14c, 14d, 14e und 14f umschlossenen Aufnahmeraum 16 auf, in dem sich (nicht näher gezeigte) Holzspänen 12 aus Zirbelholz befinden. Die Holzspäne 12 stammen aus der spanenden Bearbeitung von Zirbelholzstücken.
Der Aufnahmeraum 16 weist zwei sich gegenüberliegende Außenwände 14e und 14f auf, die als Holzwände ausgebildet sind. Die übrigen vier Außenwände 14a, 14b, 14c und 14d bestehen aus einem Geflecht 20, das diffusionsoffene Teilbereiche 18a, 18b, 18c und 18d zum Entweichen von aus den Holzspänen 12 austretenden Duftstoffen bildet.
Die sich gegenüberliegenden Holzwände 14e und14f sind durch vier an den Kantenbereichen angeordnete (nicht näher dargestellte) Streben 22a, 22b, 22c und 22d zueinander beabstandet. Die Streben 22a, 22b, 22c und 22d sind ebenfalls aus Holz gefertigt. Das Geflecht 20 ist umläufig über die Streben 22a, 22b, 22c und 22d gespannt und kann mittels einer Schraub-, Nagel-, Klammer- oder Klebeverbindung an den zwei Holzwänden 14e und14f befestigt.
Die sich gegenüberliegenden Holzwände 14e und 14f stehen an der in Figur 1 rechten Seite jeweils mit einem Wandteil 24a und 24b, das in der Figur 1 mit gestrichelten Linien zur besseren Darstellung vervollständigt ist, an einer Seite über den dazwischenliegenden Aufnahmeraum 16 hervor. Wie man der Figur 1 weiter entnehmen kann, stehen die zwei Holzwände 14e und14f darüber hinaus jeweils an allen vier Kantenbereichen über den dazwischenliegenden Aufnahmeraum 16 hervor, so dass dieser gegenüber dem Überstand an den zwischen den Holzwände 14e und14f definierten freien Flächen zurückversetzt ist. Die sich gegenüberliegenden vorstehenden Wandteile 24a und 24b weisen an der in Figur 1 rechten Seite jeweils einen Ausschnitt 26a und 26b auf, der bis zur äußeren Begrenzung des Aufnahmeraumes 16 erstreckt, wobei die Tiefe jedes der Ausschnitte 26a und 26b der Höhe des Überstandes entspricht. Wie man der Figur 1 noch weiter entnehmen kann, weisen die zwei Holzwände 14e und14f jeweils an allen vier Kantenbereichen derartige Ausschnitte auf. Die Eckbereiche sind dabei ausschnittfrei und definieren Standfüße der Haltestruktur, die sich je nach Orientierung an einer (nicht gezeigten) Unterlage oder einer (nicht gezeigten) seitlichen Fläche abstützen.
Das Geflecht 20 reicht jeweils in die Ausschnitte 26a und 26b sowie die übrigen Ausschnitte der Holzwände 14e und14f hinein und ist dort befestigt.
Die Haltestruktur 10 kann eine variable Größe aufweisen und kann z.B. Außenabmessungen von ca. 20x20x20 cm haben.
Gemäß einer (nicht gezeigten) alternativen Ausführungsform können die Außenwände 14e und 14f selbst diffusionsoffene Teilbereiche aufweisen, indem Löcher vorgesehen sind. Die Figur 2 zeigt in schematischer und perspektivischer Frontalansicht eine Ausführungsform einer auf einer Aufstellfläche 42, der Bodenfläche eines Wohnraumes, aufgestellten Regalanordnung 40, die drei durch im Wesentlichen waagrechte Regalböden 46a, 46b und 46c definierte, übereinander angeordnete Regalfächer 44a, 44b, 44c aufweist.
Jeweils zwei sich in einem Abstand a zueinander gegenüberliegend angeordnete Regalböden 46a und 46b, 46b und 46c definieren jeweils ein dazwischenliegendes Regalfach 44a und 44b.
Zwischen den zueinander gegenüberliegend angeordneten Regalböden 46a, 46b und 46c sind in einem rechten Winkel α zu diesen jeweils drei Unterteilungsböden 48a, 48b und 48c angeordnet, welche das Regalfach 44a in vier Teilfächer teilen, von denen die in Figur 2 gezeigten mittleren zwei Teilfächer mit den Bezugszeichen 50c und 50d versehen sind. Die drei Unterteilungsböden 48a, 48b und 48c teilen weiterhin das Regalfach 44b in vier Teilfächer, von denen die mittleren zwei Teilfächer mit den Bezugszeichen 50a und 50b versehen sind. Die Unterteilungsböden 48a, 48b und 48c und die Regalböden 46a, 46b und 46c sind aus durchgehenden Holzbrettern gefertigt, die mittels (nicht gezeigter) Steckausschnitte ineinandergefügt sind, so dass sie eine Gitteranordnung mit einer Mehrzahl von rasterförmig angeordneten Teilfächern 50a, 50b, 50c und 50d bilden.

In alternativen (nicht gezeigten) Ausführungsformen können entweder die senkrechten Unterteilungsböden 48a, 48b und 48c aus durchgehenden Holzbrettern gefertigt sein und die waagrechten Regalböden 46a, 46b und 46c aus einzelnen kurzen Brettern bestehen, welche in die Unterteilungsböden 48a, 48b und 48c eingehängt sind und diese in einem durch die Länge der kurzen Bretter definierten Abstand festlegen. Alternativ können die waagrechten Regalböden 46a, 46b und 46c aus durchgehenden Holzbrettern gefertigt sein und die senkrechten Unterteilungsböden 48a, 48b und 48c aus einzelnen kurzen Brettern bestehen, welche die Regalböden 46a, 46b und 46c zueinander beabstanden.

Die Regalböden 46a, 46b und 46c und die Unterteilungsböden 48a, 48b und 48c weisen an ihren Kantenbereichen Ausschnitte auf. Wie in der Figur 2 gezeigt, sind an den umlaufenden Kantenbereichen 52a, 52b, 52c und 52d des Teilfachs 50a vier Ausschnitte 54a, 54b, 54c und 54d in den Regalböden 46b und 46c und den Unterteilungsböden 48a und 48b ausgebildet, die sich in Richtung des Pfeiles M, d. h. in Richtung zur Mitte der Regalböden 46b und 46c und Unterteilungsböden 48a und 48b hin erstrecken. Die Ausschnitte 54a, 54b, 54c und 54d haben die gleichen Form, aber nicht die gleiche Größe, Länge und Tiefe wie die an den Holzwänden 14e und 14f der in Figur 1 gezeigten Haltestruktur 10 ausgebildeten Ausschnitte 26a und 26b.

Die Regalanordnung 40 weist nicht nur an der Vorderseite die Ausschnitte auf, sondern auch auf der Rückseite der Regalanordnung 40 sind diese Ausschnitte ausgebildet.

Sämtliche in der Figur dargestellten Teilfächer 50a, 50b, 50c und 50d weisen dieselben Ausschnitte 54a, 54b, 54c und 54d auf, wobei die in Figur 2 gezeigten jeweiligen Kontaktbereiche 56a, 56b, 56c, 56d, 56e und 56f zwischen den Regalböden 46a, 46b und 46c und den Unterteilungsböden 48a, 48b und 48c ausschnittfrei sind. Die Kontaktbereiche 56a, 56b, 56c, 56d, 56e und 56f sind dabei durch die Bereiche definiert in denen die Regalböden 46a, 46b und 46c und die Unterteilungsböden 48a, 48b und 48c zusammengefügt sind und Kreuzungsstellen bilden, die keine Ausschnitte aufweisen.

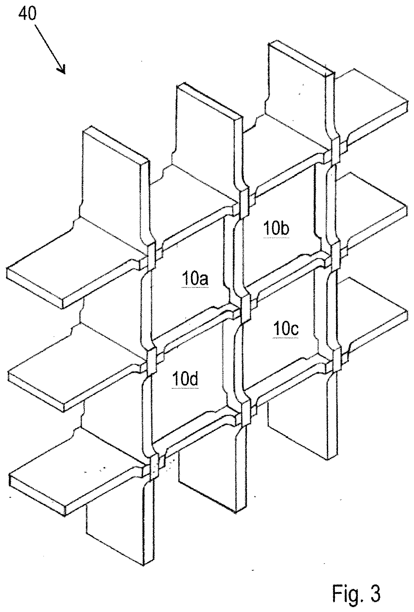

Die Figur 3 zeigt in schematischer und perspektivischer Frontalansicht die in der Figur 2 gezeigte Regalanordnung 40 mit vier in der Figur 1 gezeigten Haltestrukturen 10a, 10b, 10c und 10d, die in die vier Teilfächer 50a, 50b, 50c und 50d eingesetzt sind. In der Figur 3 ist gezeigt, dass die Haltestrukturen 10a, 10b, 10c und 10d in den Teilfächern 50a, 50b, 50c und 50d derart orientiert sind, dass eine Außenwand 14e in Richtung des Betrachters weist.

Die Maße der Teilfächer und vor allem die Größe der Haltestrukturen, sowie die Wandstärke (Tiefe) sind variabel, wobei sie z.B. quadratisch ausgebildet sein können und eine Abmessung von ca. 20x20 cm und eine Tiefe von 20 cm aufweisen können, so dass die in der Figur 1 gezeigte Haltestruktur 10 bzw. die Haltestrukturen 10a, 10b, 10c und 10d aufgenommen werden können.

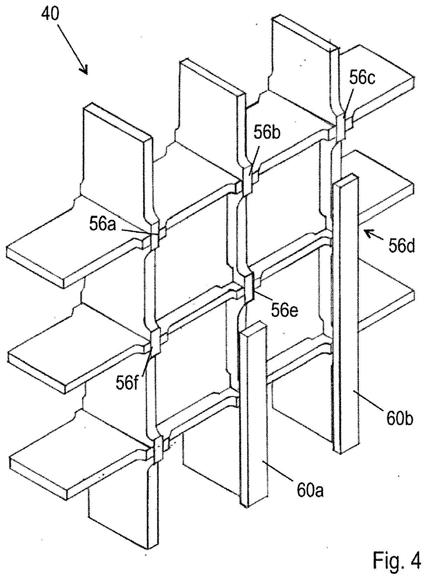

Die Figur 4 zeigt in schematischer und perspektivischer, teilweise geschnittener Frontalansicht die in der Figur 3 gezeigte Regalanordnung 40 zusätzlich mit zwei an der Vorderseite angebrachten, geschnitten dargestellten Abstandslatten 60a und 60b. Die Abstandslatte 60a ist an den Kontaktbereichen 56b und 56e und die Abstandslatte 60b ist an den Kontaktbereichen 56d und 56c durch eine (nicht gezeigte) Schraubverbindung angebracht. Die Abstandslatten 60a und 60b erstrecken sich im Wesentlichen vertikal zur Aufstellfläche 42.

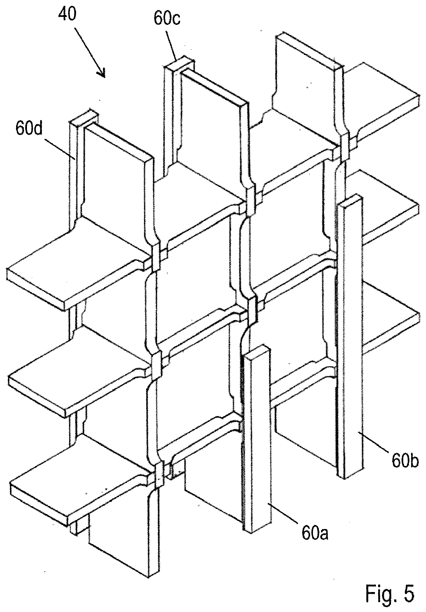

Die Figur 5 zeigt in schematischer und perspektivischer, teilweise geschnittener Frontalansicht die in der Figur 4 gezeigte Regalanordnung 40 zusätzlich mit zwei an der Rückseite an Kontaktbereichen angebrachten Abstandslatten 60c und 60d, die sich im Wesentlichen parallel zu den vorderen Abstandslatten 60a und 60b erstrecken.

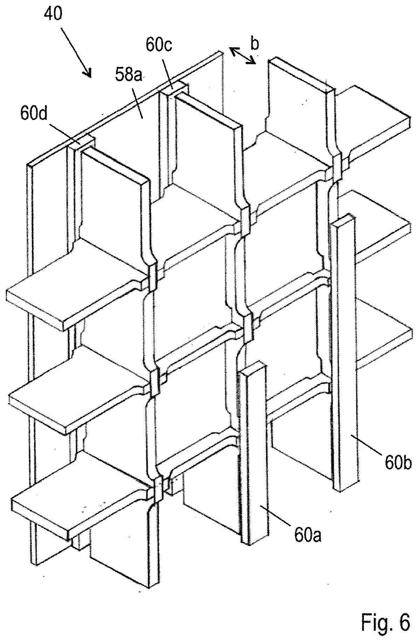

Die Figur 6 zeigt in schematischer und perspektivischer, teilweise geschnittener Frontalansicht die in der Figur 5 gezeigte Regalanordnung 40 zusätzlich mit einer an der Rückseite angebrachten Wandverkleidung 58a. Die Wandverkleidung 58a liegt an den Abstandslatten 60c und 60d an und ist an diesen durch eine (nicht gezeigte) Schraubverbindung angebracht und bildet die rückseitige Abdeckung der Regalanordnung 40. Die Abstandslatten 60c und 60d halten die Wandverkleidung 58a in einem Abstand b zu den Regalböden 46a, 46b, 46c und Unterteilungsböden 48a, 48b, 48c. Die Wandverkleidung 58a ist als eine verputzbare Leichtbauplatte ausgebildet, kann aber gemäß einer alternativen (nicht gezeigten) Ausführungsform auch aus einer Anordnung von Holzbrettern bestehen.

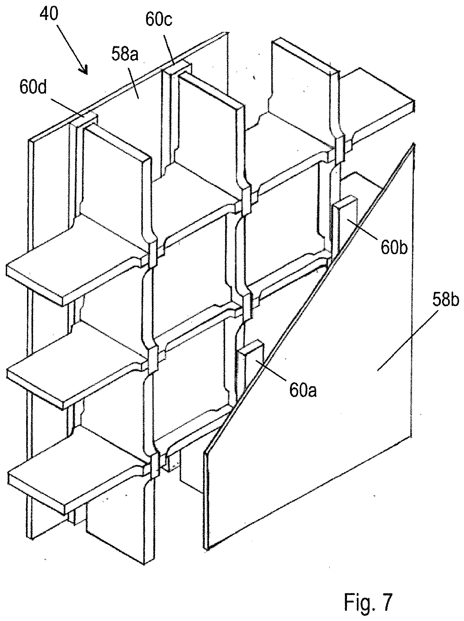

Die Figur 7 zeigt in schematischer und perspektivischer, teilweise geschnittener Frontalansicht die in der Figur 6 gezeigte Regalanordnung 40 zusätzlich mit einer an der Vorderseite an den Abstandslatten 60a und 60b angebrachten, geschnitten dargestellten Wandverkleidung 58b.

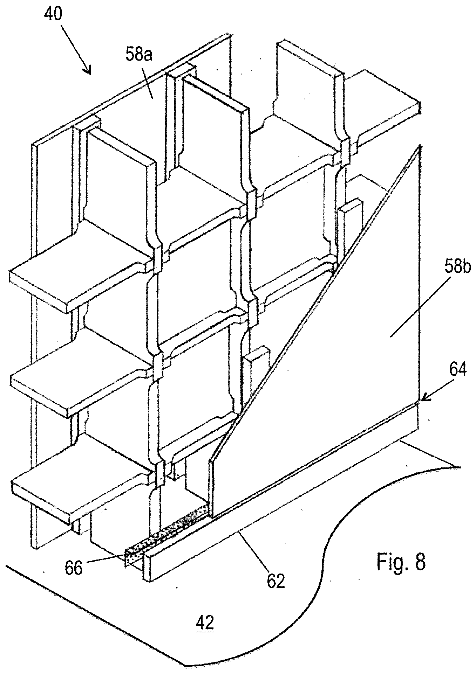

Die Figur 8 zeigt in schematischer und perspektivischer, teilweise geschnittener Frontalansicht die in der Figur 7 gezeigte Regalanordnung 40 zusätzlich mit einer an der Vorderseite unterhalb der Wandverkleidung 58b angebrachten Sockelblende 62 und einem zwischen Wandverkleidung 58b und Sockelblende 62 angebrachten Zuluftgitter 66. Die Sockelblende 62 ist zwischen der Aufstellfläche 42 und der Wandverkleidung 58b durch einen Abstandsbereich 64 beanstandet. In dem Abstandsbereich 64 ist zwischen der Sockelblende 62 und der Wandverkleidung 58b ein mit Lüftungslöchern versehenes, langgestrecktes Zuluftgitter 66 angeordnet. Das Zuluftgitter 66 weist einen L-förmigen Querschnitt auf, wobei ein Schenkel des L-förmigen Querschnitts mit einer (nicht gezeigten) Schraubverbindung an der unteren Kante der Wandverkleidung 58b angebracht ist.

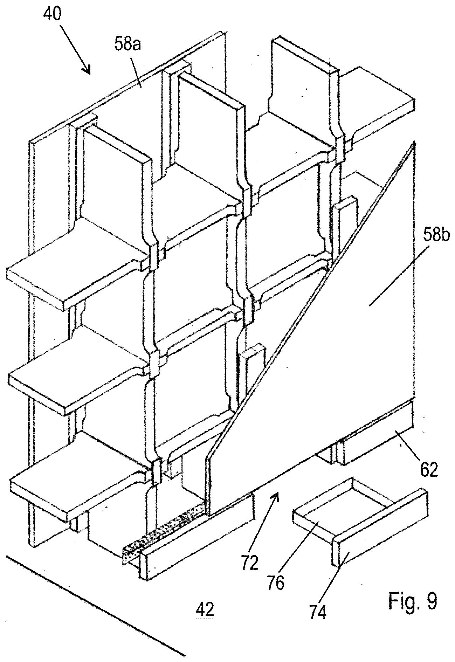

Die Figur 9 zeigt in schematischer und perspektivischer, teilweise geschnittener Frontalansicht die in der Figur 8 gezeigte Regalanordnung 40 zusätzlich mit einem an der Vorderseite an der Sockelblende 62 angeordneten Einschubfach 74 mit Wasserbehälter 76. Ein Teilbereich der Sockelblende 62 ist dazu ausgespart, um das nach Art einer Schublade ausgebildete Einschubfach 74 aufzunehmen. Das Einschubfach 74 weist eine Aufnahme für einen nach oben offenen Wasserbehälter 76 auf und ist in seinen Abmessungen auf die Beabstandung zwischen den zwei Unterteilungsböden 48b und 48c abgestimmt. Im eingeschobenen Zustand schließt das Einschubfach 74 mit der Sockelblende 62 ab und bildet einen Teil von dieser.

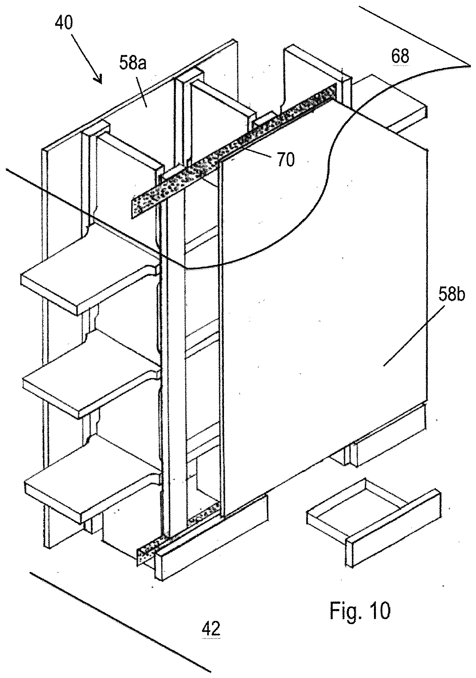

Die Figur 10 zeigt in schematischer und perspektivischer, teilweise geschnittener Frontalansicht die in der Figur 9 gezeigte Regalanordnung 40 zusätzlich mit einem an der Vorderseite oberhalb der Wandverkleidung 58b zwischen der Wandverkleidung 58b und einer Deckenfläche 68 angebrachten Abluftgitter 70. Das mit Lüftungslöchern versehene, langgestreckte Abluftgitter 70 weist einen L-förmigen Querschnitt auf, wobei ein Schenkel des L-förmigen Querschnitts mit einer (nicht gezeigten) Schraubverbindung an der oberen Kante der Wandverkleidung 58b angebracht ist und einen luftdurchlässigen Abschluss zur Deckenfläche 68 bildet. Die Wandverkleidung 58b erstreckt sich zwischen dem Zuluftgitter 66 und dem Abluftgitter 70.

In einer (nicht gezeigten) Ausführungsform kann zwischen der Wandverkleidung 58b und der Deckenfläche 68 eine von der Wandverkleidung beabstandete Blende angeordnet sein. Zwischen der Blende und der Wandverkleidung ist ein langgestrecktes Abluftgitter angebracht.

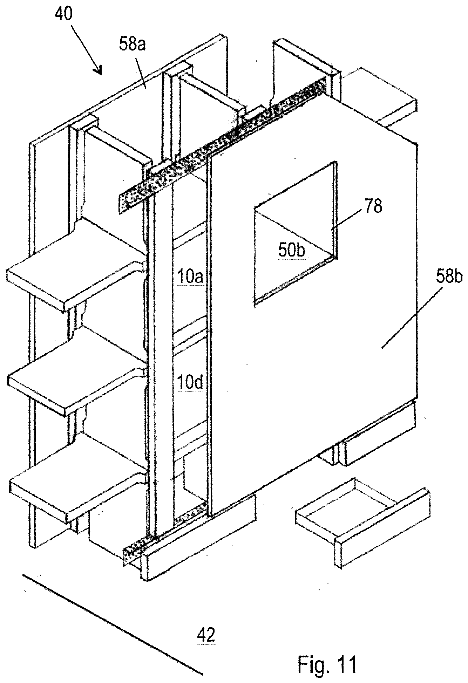

Die Figur 11 zeigt in schematischer und perspektivischer, teilweise geschnittener Frontalansicht die in der Figur 10 gezeigte Regalanordnung 40 zusätzlich mit einer an der Vorderseite in der Wandverkleidung 58b ausgebildeten Aussparung 78. In der Wandverkleidung 58b ist die Aussparung 78 ausgebildet, das Teilfach 50b der Regalanordnung 40 freizugeben.

In einer (nicht gezeigten) Ausführungsform kann die Rückseite der Regalanordnung 40 genauso wie deren Vorderseite ausgebildet sein.

## Patentansprüche

1. Haltestruktur (10a, 10b, 10c, 10d) zur Aufnahme von Holzspänen (12), mit einem durch Außenwände (14a, 14b, 14c, 14d, 14e, 14f) umschlossenen Aufnahmeraum (16) für die Holzspäne (12),
wobei die Außenwände (14a, 14b, 14c, 14d, 14e, 14f) zumindest diffusionsoffene Teilbereiche (18a, 18b, 18c, 18d) zum Entweichen von aus den Holzspänen (12) austretenden Düften / Duftstoffen aufweisen, wobei der Aufnahmeraum (16) im Wesentlichen quaderförmig aufgebaut ist und zwei sich gegenüberliegende Außenwände (14e, 14f) aufweist, die als Holzwände ausgebildet sind, und **dadurch gekennzeichnet, dass** die übrigen vier Außenwände (14a, 14b, 14c, 14d) aus einem Gitter oder Geflecht (20) bestehen,
und dass die sich gegenüberliegenden Holzwände (14e, 14f) durch vier an den Kantenbereichen angeordneten Streben (22a, 22b, 22c, 22d) zueinander beabstandet sind, wobei das Geflecht zumindest teilweise umläufig über die Streben (22a, 22b, 22c, 22d) gespannt ist.

2. Haltestruktur (10a, 10b, 10c, 10d) nach Anspruch 1,
**dadurch gekennzeichnet,**
**dass** die sich gegenüberliegenden Holzwände (14e, 14f) jeweils zumindest mit einem Wandteil (24a, 24b) an einer Seite über den dazwischenliegenden Aufnahmeraum (16) hervorstehen und die sich gegenüberliegenden vorstehenden Wandteile (24a, 24b) jeweils zumindest einen Ausschnitt (26a, 26b) aufweisen, der sich bis zur äußeren Begrenzung des Aufnahmeraumes (16) erstreckt.

3. Haltestruktur (10a, 10b, 10c, 10d) nach Anspruch 2,
**dadurch gekennzeichnet,**
**dass** das Gitter oder Geflecht (20) jeweils in die Ausschnitte (26a, 26b) hineinreicht und dort befestigt ist.

4. Wandelement (40) mit einer Regalanordnung (40) zur Aufstellung auf einer Aufstellfläche (42), mit einem oder mehreren Regalfächern (44a, 44b, 44c) und mit wenigstens einer Haltestruktur (10a, 10b, 10c, 10d) gemäß einem der Ansprüche 1 bis 3,
wobei die Haltestruktur (10a, 10b, 10c, 10d) in einem Regalfach (44a, 44b, 44c) aufgenommen ist, und wobei die Haltestruktur (10a, 10b, 10c, 10d) auswechselbar ist.

5. Wandelement (40) nach Anspruch 4,
**gekennzeichnet durch**
mindestens zwei sich in einem Abstand (a) zueinander gegenüberliegend angeordneten Regalböden (46a, 46b, 46c), die ein dazwischenliegendes Regalfach (44b, 44a) zur Aufnahme der einen oder mehrerer Haltestrukturen (10a, 10b, 10c, 10d) definieren, wobei insbesondere vorgesehen sein kann, dass zwischen den zueinander gegenüberliegend angeordneten Regalböden (46a, 46b, 46c) in einem Winkel (a) zu diesen ein oder mehrere Unterteilungsböden (48a, 48b, 48c) angeordnet sind, die das Regalfach (44a, 44b) in zwei oder mehrere Teilfächer (50a, 50b, 50c, 50d) jeweils zur Aufnahme einer oder mehrerer Haltestrukturen (10a, 10b, 10c, 10d) teilt.

6. Wandelement (40) nach Anspruch 5,
**dadurch gekennzeichnet,**
**dass** die Mehrzahl von Regalböden (46a, 46b, 46c) und Unterteilungsböden (48a, 48b, 48c) eine Gitteranordnung mit einer Mehrzahl von rasterförmig angeordneten Teilfächern (50a, 50b, 50c, 50d) bildet.

7. Wandelement (40) nach Anspruch 5 oder 6,
**dadurch gekennzeichnet,**
**dass** zumindest ein Teil der Regalböden (46a, 46b, 46c) oder Unterteilungsböden (48a, 48b, 48c) am Kantenbereich (52a, 52b, 52c, 52d) zumindest einen Ausschnitt (54a, 54b, 54c, 54d) aufweisen, der sich in Richtung Mitte (M) der Regalböden (46a, 46b, 46c) oder Unterteilungsböden (48a, 48b, 48c) erstreckt.

8. Wandelement (40) nach einem der Ansprüche 5 bis 7,
**dadurch gekennzeichnet,**
**dass** zumindest ein Teil der die Teilfächer (50a, 50b, 50c, 50d) definierenden Regalböden (46a, 46b, 46c) und Unterteilungsböden (48a, 48b, 48c) am Kantenbereich zumindest eines Teils der Teilfächer (50a, 50b, 50c, 50d) jeweils einen eingezogenen Ausschnitt (54a, 54b, 54c, 54d) aufweist, der sich vom Kantenbereich (52a, 52b, 52c, 52d) in Richtung (M) Teilfachmitte erstreckt, wobei die jeweiligen Kontaktbereiche (56a, 56b, 56c, 56d, 56e, 56f) zwischen Regalboden (46a, 46b, 46c) und Unterteilungsboden (48a, 48b, 48c) ausschnittfrei sind.

9. Wandelement (40) nach einem der Ansprüche 5 bis 8,
**dadurch gekennzeichnet,**
**dass** zumindest einseitig eine Wandverkleidung (58a, 58b) anbringbar ist, die zumindest einen Teilbereich der Regalanordnung (40) abdeckt, wobei insbesondere vorgesehen sein kann,
**dass** an zumindest einem Teil der Kontaktbereiche (56a, 56b, 56c, 56d, 56e, 56f) zwischen Regalboden (46a, 46b, 46c) und Unterteilungsboden (48a, 48b, 48c) zumindest eine Abstandslatte (60a, 60b, 60c, 60d) angebracht ist, welche die Wandverkleidung (58a, 58b) in einem Abstand (b) zu den Regalböden (46a, 46b, 46c) und Unterteilungsböden (48a, 48b, 48c) hält.

10. Wandelement (40) nach Anspruch 9,
**dadurch gekennzeichnet,**
**dass** zwischen der Aufstellfläche (42) und der Wandverkleidung (58a, 58b) eine zur Wandverkleidung (58a, 58b) beanstandete Sockelblende (62) und im Abstandsbereich (64) zwischen der Sockelblende (62) und der Wandverkleidung (58a, 58b) ein Zuluftgitter (66) angeordnet ist.

11. Wandelement (40) nach Anspruch 10,
**dadurch gekennzeichnet,**
**dass** zwischen der Wandverkleidung (58a, 58b) und einer oberhalb der Regalanordnung (40) angeordneten Deckenfläche (68) ein Abluftgitter (70) angeordnet ist, wobei sich die Wandverkleidung (58a, 58b) zwischen dem Zuluftgitter (66) und dem Abluftgitter (70) erstreckt, wobei insbesondere vorgesehen sein kann, ,
**dass** die zumindest eine Abstandslatte (60a, 60b, 60c, 60d) sich von der Aufstellfläche (42) in Richtung Deckenfläche (68) erstreckt.

12. Wandelement (40) nach einem der Ansprüche 10 oder 11,
**dadurch gekennzeichnet,**
**dass** in einem Abschnitt (72) der Sockelblende (62) ein Einschubfach (74) für einen nach oben offenen Wasserbehälter (76) ausgebildet ist.

13. Wandelement (40) nach einem der Ansprüche 9 bis 12,
**dadurch gekennzeichnet,**
**dass** in der Wandverkleidung (58a, 58b) zumindest eine Aussparung (78) ausgebildet ist, die zumindest ein Teilfach (50b) freigibt.

## Claims

1. Supporting structure (10a, 10b, 10c, 10d) for receiving wood chippings (12), with a receiving space (16) surrounded by external walls (14a, 14b, 14c, 14d, 14e, 14f) for the wood chippings (12),
wherein the external walls (14a, 14b, 14c, 14d, 14e, 14f) have at least subareas being open to diffusion (18a, 18b, 18c, 18d) for the discharge of odours / scents emerging from the wood chippings (12),
wherein the receiving space (16) is substantially build as a cuboid and has two opposing external walls (14e, 14f), which are designed as wooden walls, and **characterised in that** the other four external walls (14a, 14b, 14c, 14d) consist of a mesh or netting (20),
and that the opposing wooden walls (14e, 14f) are at a distance from one another due to four struts (22a, 22b, 22c, 22d) being arranged at the edge areas, wherein the netting is stretched at least partially circumferentially over the struts (22a, 22b, 22c, 22d).

2. Supporting structure (10a, 10b, 10c, 10d) according to claim 1,
**characterised in that**
the opposing wooden walls (14e, 14f) each protrude over the interjacent receiving space (16) at least with one wall section (24a, 24b) on one side and the opposing protruding wall sections (24a, 24b) each have at least one recess (26a, 26b), which extends to the outer boundary of the receiving space (16).

3. Supporting structure (10a, 10b, 10c, 10d) according to claim 2,
**characterised in that**
the mesh or netting (20) extends into each of the recesses (26a, 26b) and is attached there.

4. Wall element (40) with a shelf arrangement (40) for installing on an installation surface (42), with one or a number of shelves (44a, 44b, 44c) and with at least one supporting structure (10a, 10b, 10c, 10d) according to one of claims 1 to 3,
wherein the supporting structure (10a, 10b, 10c, 10d) is incorporated into a shelf (44a, 44b, 44c) and wherein the supporting structure (10a, 10b, 10c, 10d) is replaceable.

5. Wall element (40) according to claim 4,
**characterised by**
at least two shelves (46a, 46b, 46c) being arranged opposite each other at a distance (a), which define an interjacent shelf (44b, 44a) for receiving the one or number of supporting structures (10a, 10b, 10c, 10d), wherein it can be particularly provided that between the shelves (46a, 46b, 46c) being arranged opposite each other at an angle (α) to this one or a number of partitioning bases (48a, 48b, 48c) are arranged, which partition the shelf (44a, 44b) into two or more sub-compartments (50a, 50b, 50c, 50d) each for receiving one or a number of supporting structures (10a, 10b, 10c, 10d).

6. Wall element (40) according to claim 5,
**characterised in that**
the majority of shelves (46a, 46b, 46c) and partitioning bases (48a, 48b, 48c) form a lattice arrangement with a majority of sub-compartments (50a, 50b, 50c, 50d) being arranged in a grid pattern.

7. Wall element (40) according to claim 5 or 6,
**characterised in that**
at least a part of the shelves (46a, 46b, 46c) or partitioning bases (48a, 48b, 48c) have at the edge area (52a, 52b, 52c, 52d) at least one recess (54a, 54b, 54c, 54d), which extends in the direction of the centre (M) of the shelves (46a, 46b, 46c) or partitioning bases (48a, 48b, 48c).

8. Wall element (40) according to one of claims 5 to 7,
**characterised in that**
at least one part of the shelves (46a, 46b, 46c) defining the sub-compartments (50a, 50b, 50c, 50d) and partitioning bases (48a, 48b, 48c) at the edge area of at least one part of the sub-compartments (50a, 50b, 50c, 50d) has a built-in recess (54a, 54b, 54c, 54d), which extends from the edge area (52a, 52b, 52c, 52d) in the direction (M) of the sub-compartment centre, wherein the relevant contact areas (56a, 56b, 56c, 56d, 56e, 56f) between the shelves (46a, 46b, 46c) and partitioning bases (48a, 48b, 48c) are recess-free.

9. Wall element (40) according to one of claims 5 to 8,
**characterised in that**
a wall cladding (58a, 58b) is attachable at least on one side, which covers at least a sub-area of the shelf arrangement (40), wherein it can be particularly provided
that at least one spacing bar (60a, 60b, 60c, 60d) is attached to at least one part of the contact areas (56a, 56b, 56c, 56d, 56e, 56f) between the shelves (46a, 46b, 46c) and partitioning bases (48a, 48b, 48c), which holds the wall cladding (58a, 58b) at a distance (b) to the shelves (46a, 46b, 46c) and partitioning bases (48a, 48b, 48c).

10. Wall element (40) according to claim 9,
**characterised in that**
between the installation surface (42) and the wall cladding (58a, 58b) a plinth panel (62) at a distance from the wall cladding (58a, 58b) is arranged and in the space (64) between the plinth panel (62) and the wall cladding (58a, 58b) a supply air grill (66) is arranged.

11. Wall element (40) according to claim 10,
**characterised in that**
between the wall cladding (58a, 58b) and a ceiling area (68) being arranged above the shelf arrangement (40) an exhaust air grill (70) is arranged, wherein the wall cladding (58a, 58b) extends between the supply air grill (66) and the exhaust air grill (70), wherein it can be particularly provided
that the at least one spacing bar (60a, 60b, 60c, 60d) extends from the installation surface (42) in the direction of the ceiling area (68).

12. Wall element (40) according to one of the claims 10 to 11,
**characterised in that**
in a section (72) of the plinth panel (62) a drawer compartment (74) is designed for an upwardly open water container (76).

13. Wall element (40) according to one of claims 9 to 12,
**characterised in that**
in the wall cladding (58a, 58b) at least one opening (78) is designed, which releases at least one sub-compartment (50b).

## Revendications

1. Structure support (10a, 10b, 10c, 10d), destinée à loger des copeaux de bois (12), comportant un espace (16) de logement des copeaux de bois (12), entouré par des parois extérieures (14a, 14b, 14c, 14d, 14e, 14f),
les parois extérieures (14a, 14b, 14c, 14d, 14e, 14f) présentant des zones partielles (18a, 18b, 18c, 18d), au moins ouvertes à la diffusion, pour évacuer des odeurs/matières odorantes s'échappant des copeaux de bois (12),
l'espace de logement (16) ayant pour l'essentiel une configuration parallélépipédique et présentant deux parois extérieures opposées (14e, 14f) conçues comme des parois de bois, et **caractérisée en ce que** les quatre autres parois extérieures (14a, 14b, 14c, 14d) sont constituées d'un treillis ou d'un grillage (20),
et que les parois de bois opposées (14e, 14f) sont écartées l'une de l'autre par quatre étrésillons (22a, 22b, 22c, 22d), disposés dans les zones des arêtes, le grillage étant au moins partiellement tendu, sur sa périphérie, par-dessus les étrésillons (22a, 22b, 22c, 22d).

2. Structure support (10a, 10b, 10c, 10d) selon la revendication 1,
**caractérisée en ce que**
les parois de bois opposées (14e, 14f) sont chacune, au moins par une partie de paroi (24a, 24b), en saillie sur un côté par-dessus l'espace de logement intermédiaire (16), et les parties de paroi opposées en saillie (24a, 24b) présentent chacune au moins une découpe (26a, 26b), qui s'étend jusqu'à la limite extérieure de l'espace de logement (16).

3. Structure support (10a, 10b, 10c, 10d) selon la revendication 2,
**caractérisée en ce que**
le treillis ou le grillage (20) pénètrent chacun dans les découpes (26a, 26b) et y sont fixés.

4. Elément de paroi (40), comportant un arrangement de rayonnages (40), destiné à être posé sur une surface de pose (42), une ou plusieurs cases (44a, 44b, 44c) et au moins une structure support (10a, 10b, 10c, 10d) selon l'une des revendications 1 à 3,
la structure support (10a, 10b, 10c, 10d) étant logée dans une case (44a, 44b, 44c), et la structure support (10a, 10b, 10c, 10d) étant interchangeable.

5. Elément de paroi (40) selon la revendication 4,
**caractérisé par**
au moins deux étagères (46a, 46b, 46c) disposées opposées l'une à l'autre à une distance (a), qui définissent une case intermédiaire (44b, 44a) destinée à loger la ou les structures supports (10a, 10b, 10c, 10d), auquel cas on peut en particulier prévoir que, entre les étagères disposées opposées l'une à l'autre (46a, 46b, 46c), une ou plusieurs séparations (48a, 48b, 48c) sont disposées en faisant un angle (α) avec celles-ci, séparations qui subdivisent la case (44a, 44b) en deux cases partielles ou plus (50a, 50b, 50c, 50d), destinées chacune à loger une ou plusieurs structures supports (10a, 10b, 10c, 10d).

6. Elément de paroi (40) selon la revendication 5,
**caractérisé en ce que**
la pluralité d'étagères (46a, 46b, 46c) et de séparations (48a, 48b, 48c) forment un arrangement en treillis, comprenant une pluralité de cases partielles (50a, 50b, 50c, 50d) disposées en trame.

7. Elément de paroi (40) selon la revendication 5 ou 6,
**caractérisé en ce que**
au moins une partie des étagères (46a, 46b, 46c) ou des séparations (48a, 48b, 48c) présentent, dans la zone des arêtes (52a, 52b, 52c, 52d), au moins une découpe (54a, 54b, 54c, 54d), qui s'étend dans la direction du centre (M) des étagères (46a, 46b, 46c) ou des séparations (48a, 48b, 48c).

8. Elément de paroi (40) selon l'une des revendications 5 à 7,
**caractérisé en ce que**
au moins une partie des étagères (46a, 46b, 46c) et des séparations (48a, 48b, 48c) définissant les cases partielles (50a, 50b, 50c, 50d) présentent, dans la zone des arêtes d'au moins une partie des cases partielles (50a, 50b, 50c, 50d), chacune une découpe en creux (54a, 54b, 54c, 54d), qui s'étend à partir de la zone des arêtes (52a, 52b, 52c, 52d) dans la direction (M) du centre des cases partielles, les zones de contact 56a, 56b, 56c, 56d, 56e, 56f) entre chaque étagère (46a, 46b, 46c) et chaque séparation (48a, 48b, 48c) étant exemptes de découpe.

9. Elément de paroi (40) selon l'une des revendications 5 à 8,
**caractérisé en ce que**
au moins sur un côté, il est possible de rapporter un revêtement de paroi (58a, 58b), qui recouvre au moins une zone partielle de l'arrangement de rayonnages (40), auquel cas on peut en particulier prévoir
que, au moins sur une partie des zones de contact (56a, 56b, 56c, 56d, 56e, 56f) entre l'étagère (46a, 46b, 46c) et la séparation (48a, 48b, 48c), au moins une latte d'écartement (60a, 60b, 60c, 60d) est rapportée, qui maintient le revêtement de paroi (58a, 58b) à une distance (b) aux étagères (46a, 46b, 46c) et aux séparations (48a, 48b, 48c).

10. Elément de paroi (40) selon la revendication 9,
**caractérisé en ce que**
entre la surface de pose (42) et le revêtement de paroi (58a, 58b) est disposée une plinthe (62), à une certaine distance du revêtement de paroi (58a, 58b), et, dans la zone d'écartement (64) entre la plinthe (62) et le revêtement de paroi (58a, 58b) est disposée une grille d'aération (66).

11. Elément de paroi (40) selon la revendication 10,
**caractérisé en ce que**
entre le revêtement de paroi (58a, 58b) et une surface de couverture (68) disposée au-dessus de l'arrangement de rayonnages (40) est disposée une grille d'évacuation de l'air (70), le revêtement de paroi (58a, 58b) s'étendant entre la grille d'aération (66) et la grille d'évacuation de l'air (70), auquel cas on peut en particulier prévoir
que la ou les lattes d'écartement (60a, 60b, 60c, 60d) s'étendent à partir de la surface de pose (42) dans la direction de la surface de couverture (68).

12. Elément de paroi (40) selon l'une des revendications 10 ou 11,
**caractérisé en ce que**
dans une partie (72) de la plinthe (62) est réalisé un tiroir (74) destiné à un réservoir d'eau (76) ouvert vers le haut.

13. Elément de paroi (40) selon l'une des revendications 9 à 12,
**caractérisé en ce que**
dans le revêtement de paroi (58a, 58b) est réalisé au moins un évidement (78), qui dégage au moins une case partielle (50b).
